# EUROPEAN PATENT APPLICATION

(11) **EP 4 837 969 A1**
(43) Date of publication of application: **11.11.2026**
(21) Application number: 25736215.2
(22) Date of filing: 03.01.2025
(51) Int. Cl.: C12P 17/12, C12P 17/14, C12N 9/06, C12N 15/53

(54) **METHOD FOR CATALYTICALLY SYNTHESIZING SINGLE CHIRAL 2-ARYL-SUBSTITUTED NITROGEN HETEROCYCLIC DERIVATIVE BY MEANS OF IMINE REDUCTASE, AND USE THEREOF**

(30) Priority: 05.01.2024 CN 202410016532
(71) Applicant: Vividion Therapeutics, Inc., San Diego, CA 92121 (US)
(72) Inventor: TANG, Zhi, Nanjing, Jiangsu 210032 (CN); YU, Aiqin, Nanjing, Jiangsu 210032 (CN); ZHOU, Tianchi, Nanjing, Jiangsu 210032 (CN); CHEN, Jian, Nanjing, Jiangsu 210032 (CN); BAO, Xianjie, Nanjing, Jiangsu 210032 (CN); HE, Xin, Nanjing, Jiangsu 210032 (CN); ZHANG, Ye, Nanjing, Jiangsu 210032 (CN); WANG, Yuanxian, Nanjing, Jiangsu 210032 (CN)
(74) Representative: BIP Patents
(86) International application number: PCT/CN2025/070327
(87) International publication number: WO 2025/146114

(57) **Abstract**

Disclosed is a method for catalytically synthesizing a single chiral 2-aryl-substituted nitrogen-containing heterocyclic derivative by means of an imine reductase, and the use thereof. The method comprises: under the catalysis of an imine reductase, converting a compound represented by formula II into a single chiral compound represented by formula I. In formulas I and II, each R is independently selected from hydrogen, fluorine, chlorine, bromine, C1-C6 alkyl, halogenated C1-C6 alkyl, C2-C6 alkenyl, C2-C6 alkynyl, cyano, nitro, or carboxyl; X is selected from -CH2- or -O-; and n is an integer selected between 0 and 6. The imine reductase is selected from any one of or a combination of at least two of the following amino acid sequences: (1) an amino acid sequence as shown in any one of SEQ ID NOs: 2, 4, 6, 7, or 10; and (2) an amino acid sequence having imine reductase activity and having at least 90% identity to the sequence as shown in any one of SEQ ID NOs: 2, 4, 6, 7, or 10.

## Description

### Technical Field

The present invention belongs to the field of biocatalytic synthesis, and specifically relates to a method for catalytically synthesizing single chiral 2-aryl-substituted nitrogen-containing heterocyclic derivative by means of imine reductase, and the use thereof.

### Background

Chiral drugs refer to drugs composed of pharmacologically active chiral compounds. Significant differences exist between different enantiomers of chiral drugs in terms of pharmacology, pharmacokinetics, metabolism, toxicity, and immune response. Among currently used drugs, 56% are chiral molecules, wherein chiral amines and derivatives thereof constitute an important branch of chiral drugs and are structural units for numerous pharmaceutical intermediates and agrochemicals. Chiral amine drugs account for 40% of FDA-approved drugs, including drugs for neurological disorders, antihypertensives, and cardiovascular and cerebrovascular diseases.

Biological enzyme catalysts possess characteristics such as mild reaction conditions, good specificity, high stereoselectivity, and environmental benignity, and are widely used in the synthesis of various chiral compounds, including chiral amines. Examples include the application of transaminases in drugs such as sitagliptin. However, due to limitations of the reaction mechanism, transaminases are restricted to the synthesis of chiral primary amines.

Imine reductases are capable of catalyzing imine compounds to directly produce chiral secondary amines, and have advantages such as mild reaction conditions, good stereoselectivity, high conversion rate, and low production cost, thus attracting increasing attention in recent years.

Chiral 2-aryl-nitrogen heterocycles are important structural units commonly found in natural products, drug molecules, and synthetic intermediates. Functionalized chiral nitrogen-containing heterocyclic compounds have recently been demonstrated to possess various biological activities. However, the synthesis of chiral 2-aryl-nitrogen heterocycles using chemical methods requires multiple reaction steps, involves the use of chiral derivatizing reagents or metal catalysts, employs harsh conditions, causes severe pollution, makes it difficult to achieve an optical purity exceeding 98.0%, results in low yield, and has many limitations in actual large-scale production. Compared with traditional chemical synthesis methods, the use of imine reductases for catalytically synthesizing chiral 2-aryl-nitrogen heterocycles offers advantages such as low production cost, green process, and good atom economy. Some studies have also been conducted in recent years. For example, Bernhard et al. (Process Development of Enantioselective Imine Reductase-Catalyzed Syntheses of Pharmaceutically Relevant Pyrrolidines) studied the application of imine reductases in chiral 2-arylpyrrolidines. However, studies on imine reductases in other structures, such as aromatic piperidines, are still relatively limited. Therefore, searching for and screening highly active and highly selective imine reductases for the synthesis of chiral 2-aryl-nitrogen heterocycles, including aromatic piperidines, has research value and promising synthetic application prospects.

### Summary

To address the shortcomings of existing technologies, the object of the present invention is to provide a method for catalytically synthesizing single chiral 2-aryl-substituted nitrogen-containing heterocyclic derivative by means of imine reductase and the use thereof. The present invention employs a novel enzyme catalysis technology to prepare single chiral 2-aryl-nitrogen heterocycles, avoiding problems in traditional chemical synthesis routes such as the need to use chiral derivatizing reagents or metal catalysts, harsh reaction conditions, severe pollution, low chiral purity, and low yield.

To achieve the object of the present invention, the present invention adopts the following technical solution:
In a first aspect, the present invention provides a method for catalytically synthesizing single chiral 2-aryl-substituted nitrogen-containing heterocyclic derivative by means of imine reductase, the method comprising:
converting a compound represented by formula II, under catalysis by an imine reductase, into a compound represented by formula I having a single chiral form:

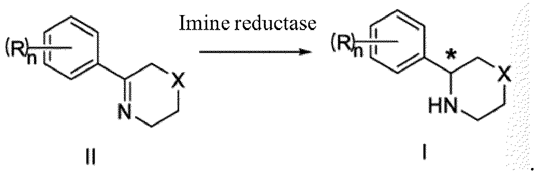

In formula I and formula II, each R is independently selected from hydrogen, fluorine, chlorine, bromine, C1-C6 alkyl, halogenated C1-C6 alkyl, C2-C6 alkenyl, C2-C6 alkynyl, cyano, nitro, or carboxyl;
X is selected from -CH₂- or -O-;
n is selected from an integer between 0 and 6; for example, n can be 0, 1, 2, 3, 4, 5, or 6, and when n is 0, R is absent;
the imine reductase is any one or a combination of at least two amino acid sequences selected from those as set forth below:
   (1) an amino acid sequence as set forth in any one of SEQ ID NO: 2, SEQ ID NO: 4, SEQ ID NO: 6, SEQ ID NO: 7, or SEQ ID NO: 10;
   (2) an amino acid sequence having at least 90% identity to any one of the sequences set forth in SEQ ID NO: 2, SEQ ID NO: 4, SEQ ID NO: 6, SEQ ID NO: 7, or SEQ ID NO: 10 and having imine reductase activity.

In the present invention, the sources of the imine reductase include *Myxococcus stipitatus*, *Mycolicibacterium mageritense*, *Burkholderia contaminans* FFH2055, *Paenibacillus mucilaginosus*, and *Luteolibacter luteus*.

Preferably, the compound represented by formula II is converted, under catalysis of any one or a combination of at least two of the imine reductases as set forth in SEQ ID NO: 2, SEQ ID NO: 6, or SEQ ID NO: 10, into the compound represented by formula I in S-configuration.

Preferably, the compound represented by formula II is converted, under catalysis of any one or a combination of two of the imine reductases as set forth in SEQ ID NO: 4 or SEQ ID NO: 8, into the compound represented by formula I in R-configuration.

Preferably, the method for catalytically synthesizing single chiral 2-aryl-substituted nitrogen-containing heterocyclic derivative by means of imine reductase comprises:
preparing a reaction system comprising the compound represented by formula II, an imine reductase enzyme powder, and a buffer solution, and carrying out a catalytic reaction to obtain the product represented by formula I;
or, preparing a reaction system comprising the compound represented by formula II, cells containing an imine reductase, and a buffer solution, and carrying out a catalytic reaction to obtain the product represented by formula I.

Preferably, the reaction system further comprises a coenzyme.

Preferably, the coenzyme is selected from any one of NAD⁺, NADH, NADP⁺, or NADPH, or a combination of at least two thereof.

Preferably, the coenzyme is NADP⁺.

Preferably, the initial reaction concentration of the coenzyme is 0.02-0.4 g/L, for example, it can be 0.02 g/L, 0.05 g/L, 0.1 g/L, 0.15 g/L, 0.2 g/L, 0.25 g/L, 0.3 g/L, 0.35 g/L, or 0.4 g/L, preferably 0.05-0.1 g/L, for example, it can be 0.05 g/L, 0.06 g/L, 0.07 g/L, 0.08 g/L, 0.09 g/L, or 0.1 g/L.

Preferably, the reaction system further comprises a co-substrate selected from any one of isopropanol, glucose, or ammonium formate, or a combination of at least two thereof.

Preferably, the co-substrate is glucose.

Preferably, the initial reaction concentration of the co-substrate is 20-50 g/L, for example, it can be 20 g/L, 25 g/L, 30 g/L, 35 g/L, 40 g/L, 45 g/L, or 50 g/L, etc., preferably 30-40 g/L.

Preferably, the reaction system further comprises an enzyme for coenzyme regeneration selected from any one of alcohol dehydrogenase, formate dehydrogenase, or glucose dehydrogenase, or a combination of at least two thereof.

Preferably, the enzyme for coenzyme regeneration is glucose dehydrogenase.

Preferably, the initial reaction concentration of the enzyme for coenzyme regeneration is 0.02-0.4 g/L, for example, it can be 0.02 g/L, 0.05 g/L, 0.1 g/L, 0.15 g/L, 0.2 g/L, 0.25 g/L, 0.3 g/L, 0.35 g/L, or 0.4 g/L, etc., preferably 0.05-0.1 g/L.

Preferably, the initial reaction concentration of the compound represented by formula II in the reaction system is 1-100 g/L, for example, it can be 1 g/L, 10 g/L, 20 g/L, 30 g/L, 40 g/L, 50 g/L, 60 g/L, 70 g/L, 80 g/L, 90 g/L, or 100 g/L, etc., preferably 20-80 g/L, more preferably 30-50 g/L.

Preferably, the initial reaction concentration of the imine reductase enzyme powder in the reaction system is 1-20 g/L, for example, it can be 1 g/L, 2 g/L, 4 g/L, 6 g/L, 8 g/L, 10 g/L, 12 g/L, 14 g/L, 16 g/L, 18 g/L, or 20 g/L, etc., preferably 5-10 g/L.

Preferably, the initial reaction concentration of the cells containing the imine reductase in the reaction system is 5-100 g/L, for example, it can be 5 g/L, 10 g/L, 20 g/L, 30 g/L, 40 g/L, 50 g/L, 60 g/L, 70 g/L, 80 g/L, 90 g/L, or 100 g/L, etc., preferably 20-80 g/L, more preferably 40-60 g/L.

In the present invention, the reaction system is a buffered saline solution system, and the pH of the reaction system is controlled by a buffer solution.

Preferably, the buffer solution is selected from any one of a potassium phosphate buffer, a tris(hydroxymethyl)aminomethane-hydrochloride buffer, an ammonium chloride buffer, an ammonium formate buffer, or an ammonium acetate buffer, or a combination of at least two thereof.

Commonly used buffer solutions include, but are not limited to, potassium phosphate buffer, tris(hydroxymethyl)aminomethane-hydrochloride (Tris-HCl), ammonium chloride (NH₄Cl), ammonium formate (HCOONH₄), or ammonium acetate (CH₃COONH₄) buffer.

Preferably, the buffer solution is a potassium phosphate buffer.

Preferably, the concentration of potassium phosphate in the potassium phosphate buffer is 20-200 mM, for example, it can be 20 mM, 25 mM, 50 mM, 75 mM, 100 mM, 125 mM, 150 mM, 175 mM, or 200 mM, etc., preferably 90-100 mM.

Preferably, the reaction system further comprises a co-solvent selected from any one of dimethyl sulfoxide, methanol, ethanol, isopropanol, or acetone, or a combination of at least two thereof.

Preferably, the co-solvent is dimethyl sulfoxide.

In the present invention, the co-solvent used is miscible with water to further increase the solubility of the substrate.

In a specific embodiment of the present invention, the technical solution adopted by the present invention is as shown below:

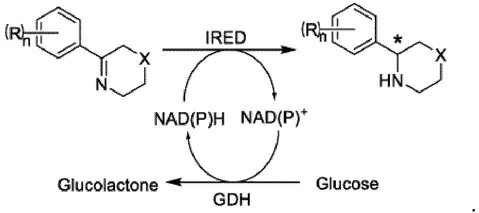

Preferably, the reaction time of the catalytic reaction is 12-36 hours, for example, it can be 12, 16, 20, 24, 28, 32, or 36, etc., preferably 20-24 hours.

Preferably, the reaction temperature of the catalytic reaction is 25-40°C, for example, it can be 25°C, 27°C, 29°C, 31°C, 33°C, 35°C, 37°C, 39°C, or 40°C, etc., preferably 30-37°C.

Preferably, the pH of the reaction system of the catalytic reaction is pH 6.0-8.0, for example, it can be pH 6.0, 6.5, 7.0, 7.5, or 8.0, etc., preferably pH 7.0-7.5.

Preferably, the imine reductase enzyme powder is obtained by fermentation of a genetically engineered microorganism.

Preferably, the genetically engineered microorganism is a genetically engineered *Escherichia coli* or yeast, preferably *Escherichia coli*.

Preferably, the genetic engineering comprises introducing a nucleotide encoding the imine reductase into *Escherichia coli* or yeast, and expressing the imine reductase in *Escherichia coli* or yeast.

Preferably, the nucleotide encoding the imine reductase is any one or a combination of at least two sequences selected from thoseas set forth below:
(1) a nucleotide sequence as set forth in any one of SEQ ID NO: 1, SEQ ID NO: 3, SEQ ID NO: 5, SEQ ID NO: 7, or SEQ ID NO: 9;
(2) a nucleotide sequence having at least 90% identity to any one of the sequences set forth in SEQ ID NO: 1, SEQ ID NO: 3, SEQ ID NO: 5, SEQ ID NO: 7, or SEQ ID NO: 9 and encoding an imine reductase.

Taking (S)-2-(4-fluorophenyl)-piperidine 5(a) and (R)-3-(3-bromo-5-chlorophenyl)-morpholine 10(b) prepared by the present invention as examples, the conversion rate is not less than 99%, and the enantiomeric excess value is not less than 99.0%. The present invention employs a novel enzyme catalysis technology to achieve the manufacture of single chiral 2-aryl-nitrogen heterocycle, using a selected imine reductase with a specific amino acid sequence to carry out the catalytic reaction. A coenzyme can also be added to the reaction as an electron transfer agent, and glucose dehydrogenase can also be used in combination to achieve coenzyme recycling. This technical route has simple steps, high conversion rate, good selectivity, is green and environmentally friendly, and has potential for industrial production.

In a second aspect, the present invention provides the use of the method for catalytically synthesizing single chiral 2-aryl-substituted nitrogen-containing heterocyclic derivative by means of imine reductase according to the first aspect in the manufacture of a chiral drug.

The numerical ranges recited in the present invention include not only the specific point values listed above but also any other point values falling within the above-mentioned numerical ranges that are not explicitly listed. Due to space limitations and for the sake of brevity, the present invention does not exhaustively list the specific point values encompassed by such ranges.

Compared with the prior art, the present invention has the following beneficial effects: a novel enzyme catalysis technology is provided, and the manufacture of single chiral 2-aryl-nitrogen heterocycle is achieved, which avoids problems in traditional chemical synthesis routes such as the need to use chiral derivatizing reagents or metal catalysts, harsh reaction conditions, severe pollution, low chiral purity, and low yield.

### Brief Description of the Drawings

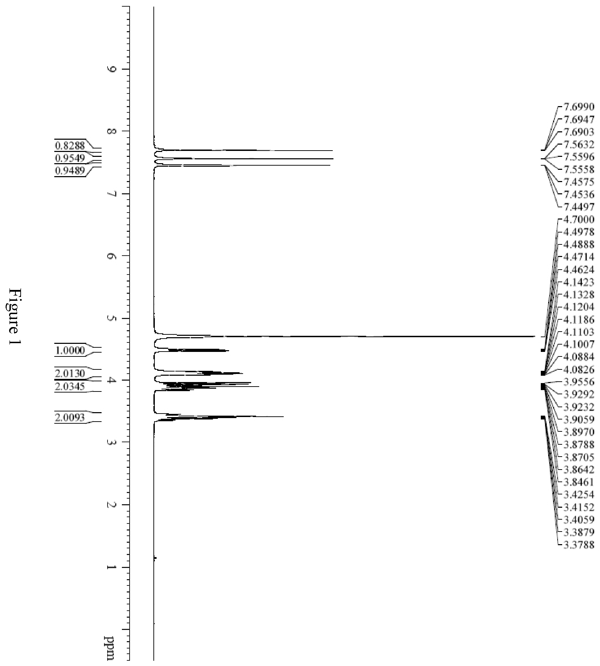

Figure 1 is a nuclear magnetic resonance spectrum of compound 10(b).

### Detailed Description

The technical solution of the present invention is further illustrated with reference to specific embodiments below. It should be understood by those skilled in the art that the examples are merely to aid in understanding the present invention and should not be construed as specifically limiting the present invention.

If no specific techniques or conditions are specified in the examples, the techniques or conditions described in the literature in the field or the product instructions are used. If no manufacturer is specified for the reagents or instruments used, they are all conventional products that can be purchased through regular channels.

### Example 1: Synthesis and Expression of Imine Reductase

The coding genes of wild-type imine reductases derived from *Luteolibacter luteus*, *Burkholderia contaminans* FFH2055, *Paenibacillus mucilaginosus*, *Mycolicibacterium mageritense*, and *Myxococcus stipitatus* were codon-optimized, and the corresponding DNA sequences were synthesized.
SEQ ID NO: 1 is the nucleotide sequence encoding the imine reductase derived from *Myxococcus stipitatus*;
SEQ ID NO: 2 is the amino acid sequence of the imine reductase derived from *Myxococcus stipitatus*;
SEQ ID NO: 3 is the nucleotide sequence encoding the imine reductase derived from *Mycolicibacterium mageritense*;
SEQ ID NO: 4 is the amino acid sequence of the imine reductase derived from *Mycolicibacterium mageritense*;
SEQ ID NO: 5 is the nucleotide sequence encoding the imine reductase derived from *Burkholderia contaminans* FFH2055;
SEQ ID NO: 6 is the amino acid sequence of the imine reductase derived from *Burkholderia contaminans* FFH2055;
SEQ ID NO: 7 is the nucleotide sequence encoding the imine reductase derived from *Paenibacillus mucilaginosus*;
SEQ ID NO: 8 is the amino acid sequence of the imine reductase derived from *Paenibacillus mucilaginosus*;
SEQ ID NO: 9 is the nucleotide sequence encoding the imine reductase derived from *Luteolibacter luteus*; and
SEQ ID NO: 10 is the amino acid sequence of the imine reductase derived from *Luteolibacter luteus*.

Each gene fragment was then ligated into the plasmid pET28a(+). Subsequently, the recombinant plasmid was transformed into *Escherichia coli* BL21(DE3) by chemical transformation or electroporation, and the bacterial liquid was spread onto LB agar plates containing 50 µg/mL kanamycin, and incubated at 37°C overnight. Transformants were selected for sequencing verification, and the verified transformants were named LlIR, BcIR, PmIR, MmIR, and MsIR respectively.

The above recombinant bacteria were inoculated into 5 mL of LB liquid medium containing 50 µg/mL kanamycin, and incubated at 37°C overnight. 2 mL of the bacterial liquid was inoculated into 200 mL of LB liquid medium containing 50 µg/mL kanamycin, and incubated at 37°C, 200 rpm for 3 hours. Then IPTG was added to a final concentration of 50 mM, and induction was carried out at 25°C, 200 rpm for 16 hours. Centrifugation was performed at 9000 rpm, 4°C for 10 minutes to collect the bacterial cells, thereby obtaining wet cells containing the corresponding imine reductase. The collected wet cells were freeze-dried at -80°C to obtain the corresponding imine reductase enzyme powders, designated as *Ll*IR, *Bc*IR, *Pm*IR, *Mm*IR, and *Ms*IR respectively.

### Example 2: Synthesis of 6-(4-fluorophenyl)-2,3,4,5-tetrahydropyridine

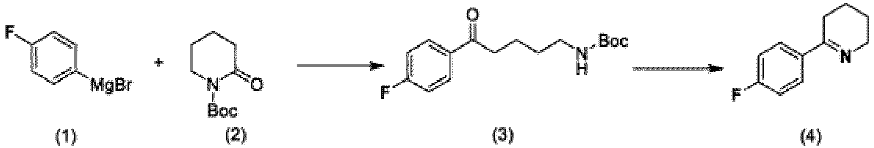

In a four-necked flask equipped with magnetic stirrer, 5.97 g (30 mmol) of lactam 2 was dissolved in THF (100 mL). The solution was cooled to -30°C, and 6.57 g (33 mmol) of Grignard reagent 1 was added dropwise. The mixture was stirred for 1 h at this temperature. The reaction solution was quenched with 1 M HCl (100 mL), MTBE was added for extraction (100 mL × 3). The organic phase was washed with saturated brine, dried over anhydrous MgSO₄, filtered, and distilled under reduced pressure to obtain 7.96 g (27 mmol) of N-Boc aryl ketone 3. 7.96 g (27 mmol) of N-Boc aryl ketone 3 was dissolved in DCM (15 mL). The solution was cooled to 0°C. TFA (15 mL) was added dropwise. The mixture was stirred for 2 h at this temperature. 30% NaOH was added dropwise to adjust the pH to 10-11. MTBE was added for extraction (100 mL × 3). The organic phase was washed with saturated brine, dried over anhydrous MgSO₄, filtered, and distilled under reduced pressure to obtain 4.3 g of aryl cyclic imine 4, with a yield of 81%.

### Example 3: Small-Scale Experiment of 6-(4-fluorophenyl)-2,3,4,5-tetrahydropyridine

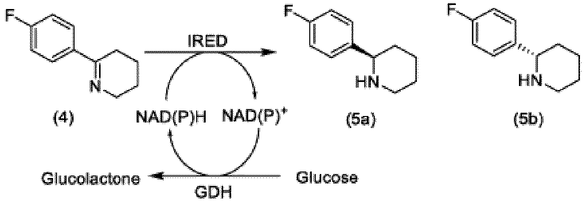

10.0 mg of each imine reductase enzyme powder (*Ll*IR, *Bc*IR, *Pm*IR, *Mm*IR, *Ms*IR) was weighed into corresponding 5 mL centrifuge tubes. 2 mg of GDH, 20 mg of glucose, 2 mg of NADP⁺, 20 mg of Compound 4 (6-(4-fluorophenyl)-2,3,4,5-tetrahydropyridine) and 20 µL of DMSO solution were respectively added to 0.1 M phosphate buffer (pH 7.0), and the volume was adjusted to 1.0 mL. Subsequently, the mixture was placed in a shaker at 30°C for reaction for 24 hours, and then detected by UPLC and chiral HPLC. The conversion rate and ee value are shown in Table 1 below.

| Enzyme | Source | NCBI | Conv(%) | ee(%) |
|---|---|---|---|---|
| *Ms*IR | *Myxococcus stipitatus* | WP_015347361.1 | >99.0% | >99.0% (*S*) |
| *Mm*IR | *Mycolicibacterium mageritense* | WP_036431480.1 | >99.0% | 97.2% (*R*) |
| *Bc*IR | *Burkholderia contaminans* FFH2055 | KKL35406.1 | >99.0% | >99.0% (*S*) |
| *Pm*IR | *Paenibacillus mucilaginosus* | WP_016362380.1 | >98.7% | 96.5% (*R*) |
| *Ll*IR | *Luteolibacter luteus* | WP_205760981.1 | >99.0% | >99.0% (*S*) |

### Example 4: Gram-Scale Experiment of 6-(4-fluorophenyl)-2,3,4,5-tetrahydropyridine

To a 50 mL four-necked flask were added 1.0 g of starting material 6-(4-fluorophenyl)-2,3,4,5-tetrahydropyridine and 2% v.v⁻¹ DMSO, 0.05 g of NADP⁺, 0.05 g of GDH, 2.0 g of glucose, and 0.1 M phosphate buffer (pH 7.0) to make up the volume to 30 mL. The mixture was stirred to initiate the reaction, a constant-temperature water bath was used to control the temperature at 30°C, a titrator was used to control the pH at 7.0 with 3 M sodium carbonate solution, and finally 1.5 g of wet cells, *Bc*IR, were added to the reaction system. After 24 hours, the detection showed Conv(%) > 99.0% and ee > 99.0% (S).

### Example 5: Synthesis of 5-(3-bromo-5-chlorophenyl)-3,6-dihydro-1,4-oxazine

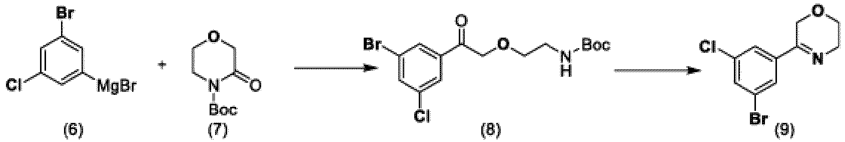

In a four-necked flask equipped with magnetic stirrer, 6.03 g (30 mmol) of lactam 7 was dissolved in THF (100 mL). The solution was cooled to -30°C. 9.70 g (33 mmol) of Grignard reagent 6 was added dropwise. The mixture was stirred for 1 h at this temperature. The reaction solution was quenched with 1 M HCl (100 mL). MTBE was added for extraction (100 mL × 3). The organic phase was washed with saturated brine, dried over anhydrous MgSO₄, filtered, and distilled under reduced pressure to obtain 9.97 g (25.5 mmol) of N-Boc aryl ketone 8. 9.97 g (25.5 mmol) of N-Boc aryl ketone 8 was dissolved in DCM (15 mL). The solution was cooled to 0°C. TFA (15 mL) was added dropwise. The mixture was stirred for 2 h at this temperature. 30% NaOH was added dropwise to adjust the pH to 10-11, MTBE was added for extraction (100 mL × 3), the organic phase was washed with saturated brine, dried over anhydrous MgSO₄, filtered, and distilled under reduced pressure to obtain 5.57 g of aryl cyclic imine 9, with a yield of 68%.

### Example 6: Small-Scale Experiment of 5-(3-bromo-5-chlorophenyl)-3,6-dihydro-1,4-oxazine

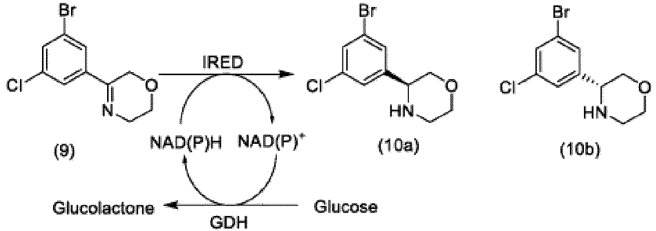

10.0 mg of each imine reductase (*Ll*IR, *Bc*IR, *Pm*IR, *Mm*IR, *Ms*IR) was weighed into corresponding 5 mL centrifuge tubes. 2 mg of GDH, 20 mg of glucose, 2 mg of NADP⁺, 20 mg of 5-(3-bromo-5-chlorophenyl)-3,6-dihydro-1,4-oxazine and 20 µL of DMSO solution were respectively added to 0.1 M phosphate buffer (pH 7.0), and the volume was adjusted to 1.0 mL. Subsequently, the mixture was placed in a shaker at 30°C for reaction for 24 hours, and then the conversion rate and chirality were detected. The results are shown in Table 2 below:

| Enzyme No. | Source | NCBI | Conv(%) | ee(%) |
|---|---|---|---|---|
| *MsIR* | *Myxococcus stipitatus* | WP_015347361.1 | >99.0% | >99.0% (*S*) |
| *MmIR* | *Mycolicibacterium mageritense* | WP_036431480.1 | >99.0% | 95.7% (*R*) |
| *BcIR* | *Burkholderia contaminans* FFH2055 | KKL35406.1 | >99.0% | >99.0% (*S*) |
| *PmIR* | *Paenibacillus mucilaginosus* | WP_016362380.1 | >98.1% | >99.0% (*R*) |
| *Ll*IR | *Luteolibacter luteus* | WP 205760981.1 | >99.0% | >99.0% (*R*) |

### Example 7: Gram-Scale Experiment of 5-(3-bromo-5-chlorophenyl)-3,6-dihydro-1,4-oxazine

To a 50 mL four-necked flask were added 1.0 g of starting material 5-(3-bromo-5-chlorophenyl)-3,6-dihydro-1,4-oxazine and 2% v.v⁻¹ DMSO, 0.05 g of NADP⁺, 0.05 g of GDH, 2.0 g of glucose, and 0.1 M phosphate buffer (pH 7.0) to make up the volume to 30 mL. The mixture was stirred to initiate the reaction, a constant-temperature water bath was used to control the temperature at 30°C, a titrator was used to control the pH at 7.0 with 3 M sodium carbonate solution, and finally 1.5 g of wet cells, *Ll*IR, were added to the reaction system. After 24 hours, the detection showed Conv(%) > 99.0% and ee > 99.0% (*R*).

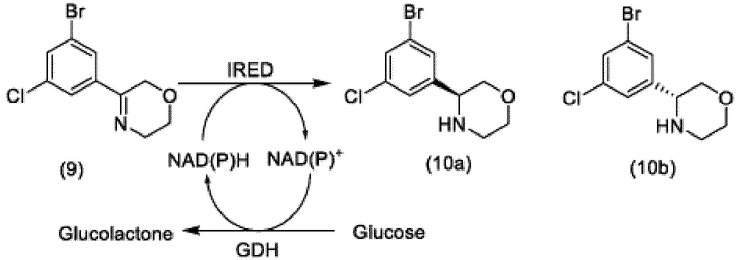

### Example 8: Ten-Gram Scale Experiment of 5-(3-bromo-5-chlorophenyl)-3,6-dihydro-1,4-oxazine

To a 500 mL four-necked flask were added 10.0 g of starting material 5-(3-bromo-5-chlorophenyl)-3,6-dihydro-1,4-oxazine and 2% v.v⁻¹ DMSO, 0.5 g of NADP⁺, 0.5 g of GDH, 20.0 g of glucose, and 0.1 M phosphate buffer (pH 7.0) to make up the volume to 300 mL. The mixture was stirred to initiate the reaction, a constant-temperature water bath was used to control the temperature at 30°C, a titrator was used to control the pH at 7.0 with 3 M sodium carbonate solution, and finally 15.0 g of wet cells, *Ll*IR, were added to the reaction system. After 24 hours, the detection showed Conv(%) > 99.0% and ee > 99.0% (R).

The applicant declares that the above descriptions are merely specific embodiments of the present invention, but the protection scope of the present invention is not limited thereto. It should be understood by those skilled in the art that any changes or substitutions that can be easily conceived by any person skilled in the art within the technical scope disclosed by the present invention fall within the protection scope and disclosure scope of the present invention.

## Claims

1. A method for catalytically synthesizing single chiral 2-aryl-substituted nitrogen-containing heterocyclic derivative by means of imine reductase, comprising:
converting a compound represented by formula II into a compound represented by formula I having a single chiral form, under catalysis of an imine reductase: 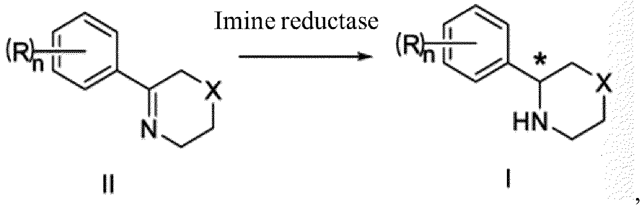
wherein, in formula I and formula II, each R is independently selected from hydrogen, fluorine, chlorine, bromine, C1-C6 alkyl, halogenated C1-C6 alkyl, C2-C6 alkenyl, C2-C6 alkynyl, cyano, nitro, or carboxyl;
X is selected from -CH₂- or -O-;
n is selected from an integer between 0 and 6;
the imine reductase is any one or a combination of at least two amino acid sequences selected from those as set forth below:
(1) an amino acid sequence as set forth in any one of SEQ ID NO: 2, SEQ ID NO: 4, SEQ ID NO: 6, SEQ ID NO: 7, or SEQ ID NO: 10;
(2) an amino acid sequence having at least 90% identity to any one of the sequences as set forth in SEQ ID NO: 2, SEQ ID NO: 4, SEQ ID NO: 6, SEQ ID NO: 7, or SEQ ID NO: 10 and having imine reductase activity.

2. The method for catalytically synthesizing single chiral 2-aryl-substituted nitrogen-containing heterocyclic derivative by means of imine reductase according to claim 1, **characterized in that** the compound represented by formula II is converted, under catalysis of any one or a combination of at least two of the imine reductases as set forth in SEQ ID NO: 2, SEQ ID NO: 6, or SEQ ID NO: 10, into the compound represented by formula I in S-configuration;
preferably, the compound represented by formula II is converted, under catalysis of any one or a combination of two of the imine reductases as set forth in any one of SEQ ID NO: 4 or SEQ ID NO: 8, into the compound represented by formula I in R-configuration.

3. The method for catalytically synthesizing single chiral 2-aryl-substituted nitrogen-containing heterocyclic derivative by means of imine reductase according to claim 1 or 2, **characterized in that** the method comprises:
preparing a reaction system comprising the compound represented by formula II, an imine reductase enzyme powder and a buffer solution, and carrying out a catalytic reaction to obtain the product represented by formula I;
or, preparing a reaction system comprising the compound represented by formula II, cells containing an imine reductase, and a buffer solution, and carrying out a catalytic reaction to obtain the product represented by formula I.

4. The method for catalytically synthesizing single chiral 2-aryl-substituted nitrogen-containing heterocyclic derivative by means of imine reductase according to claim 3, **characterized in that** the reaction system further comprises a coenzyme;
preferably, the coenzyme is selected from any one of NAD⁺, NADH, NADP⁺, or NADPH, or a combination of at least two thereof;
preferably, the coenzyme is NADP⁺;
preferably, the initial reaction concentration of the coenzyme is 0.02-0.4 g/L, preferably 0.05-0.1 g/L.

5. The method for catalytically synthesizing single chiral 2-aryl-substituted nitrogen-containing heterocyclic derivative by means of imine reductase according to claim 3 or 4, **characterized in that** the reaction system further comprises a co-substrate selected from any one of isopropanol, glucose, or ammonium formate, or a combination of at least two thereof;
preferably, the co-substrate is glucose;
preferably, the initial reaction concentration of the co-substrate is 20-50 g/L, preferably 30-40 g/L;
preferably, the reaction system further comprises an enzyme for coenzyme regeneration, selected from any one of alcohol dehydrogenase, formate dehydrogenase, or glucose dehydrogenase, or a combination of at least two thereof;
preferably, the enzyme for coenzyme regeneration is glucose dehydrogenase;
preferably, the initial reaction concentration of the enzyme for coenzyme regeneration is 0.02-0.4 g/L, preferably 0.05-0.1 g/L.

6. The method for catalytically synthesizing single chiral 2-aryl-substituted nitrogen-containing heterocyclic derivative by means of imine reductase according to any one of claims 3-5, **characterized in that** the initial reaction concentration of the compound represented by formula II in the reaction system is 1-100 g/L, preferably 20-80 g/L, more preferably 30-50 g/L;
preferably, the initial reaction concentration of the imine reductase enzyme powder in the reaction system is 1-20 g/L, preferably 5-10 g/L;
preferably, the initial reaction concentration of the cells containing the imine reductase in the reaction system is 5-100 g/L, preferably 20-80 g/L, more preferably 40-60 g/L.

7. The method for catalytically synthesizing single chiral 2-aryl-substituted nitrogen-containing heterocyclic derivative by means of imine reductase according to any one of claims 3-6, **characterized in that** the buffer solution is selected from any one of a potassium phosphate buffer, a tris(hydroxymethyl)aminomethane-hydrochloride buffer, an ammonium chloride buffer, an ammonium formate buffer, or an ammonium acetate buffer, or a combination of at least two thereof;
preferably, the buffer solution is a potassium phosphate buffer;
preferably, the concentration of potassium phosphate in the potassium phosphate buffer is 20-200 mM, preferably 90-100 mM;
preferably, the reaction system further comprises a co-solvent selected from any one of dimethyl sulfoxide, methanol, ethanol, isopropanol, or acetone, or a combination of at least two thereof;
preferably, the co-solvent is dimethyl sulfoxide.

8. The method for catalytically synthesizing single chiral 2-aryl-substituted nitrogen-containing heterocyclic derivative by means of imine reductase according to any one of claims 3-7, **characterized in that** the reaction time of the catalytic reaction is 12-36 hours, preferably 20-24 hours;
preferably, the reaction temperature of the catalytic reaction is 25-40°C, preferably 30-37°C;
preferably, the pH of the reaction system of the catalytic reaction is 6.0-8.0, preferably 7.0-7.5.

9. The method for catalytically synthesizing single chiral 2-aryl-substituted nitrogen-containing heterocyclic derivative by means of imine reductase according to any one of claims 3-8, **characterized in that** the imine reductase enzyme powder is obtained by fermentation of a genetically engineered microorganism;
preferably, the genetically engineered microorganism is a genetically engineered *Escherichia coli* or yeast, preferably *Escherichia coli*;
preferably, the genetic engineering comprises introducing a nucleotide encoding the imine reductase into *Escherichia coli* or yeast, and expressing the imine reductase in *Escherichia coli* or yeast;
preferably, the nucleotide encoding the imine reductase is any one or a combination of at least two sequences selected from those as set forth below:
(I) a nucleotide sequence as set forth in any one of SEQ ID NO: 1, SEQ ID NO: 3, SEQ ID NO: 5, SEQ ID NO: 7, or SEQ ID NO: 9;
(II) a nucleotide sequence having at least 90% identity to any one of the sequences set forth in SEQ ID NO: 1, SEQ ID NO: 3, SEQ ID NO: 5, SEQ ID NO: 7, or SEQ ID NO: 9 and encoding an imine reductase.

10. Use of the method for catalytically synthesizing single chiral 2-aryl-substituted nitrogen-containing heterocyclic derivative by means of imine reductase according to any one of claims 1-9 in the manufacture of a chiral drug.
